# DEMANDE DE BREVET EUROPEEN

(11) **EP 4 413 868 A2**
(43) Date de publication de la demande: **14.08.2024**
(21) Numéro de dépôt: 24185836.4
(22) Date de dépôt: 14.05.2019
(51) Int. Cl.: A23K 50/10

(54) **PROBIOTIQUE POUR VOLAILLE**

(30) Priorité: 15.05.2018 FR 1854046
(62) Demande divisionnaire de: 19737159.4
(71) Demandeur: Lesaffre et Compagnie, 75001 Paris (FR)
(72) Inventeur: AUCLAIR, Eric, 59710 AVELIN (FR); JULIEN, Christine, 31360 SEPX (FR); MARDEN, Jean-Philippe, 31670 LABEGE (FR)
(74) Mandataire: Plasseraud IP

(57) **Abrégé**

La présente invention se rapporte à des levures actives adaptées à des acides organiques faibles et à leur utilisation chez la volaille ou chez les ruminants. L'invention se rapporte également à un probiotique pour volaille ou ruminant comprenant une levure active adaptée aux acides organiques faibles.

La présente invention concerne également l'utilisation d'une telle levure pour améliorer les performances zootechniques de la volaille et son utilisation dans un probiotique et/ou un aliment pour volaille.

Elle concerne encore l'utilisation d'une telle levure pour améliorer les paramètres physico-chimiques et fermentaires du rumen des ruminants, et son utilisation dans un probiotique et/ou un aliment pour ruminant.

L'invention concerne encore une telle levure pour une utilisation dans la prévention et/ou le traitement des troubles digestifs du ruminant et en particulier dans la prévention de l'acidose chez le ruminant.

## Description

### DOMAINE DE L'INVENTION

La présente invention concerne le domaine des probiotiques pour volaille ou ruminant. Elle concerne plus particulièrement un probiotique et un aliment pour volaille ou ruminant le comprenant, l'utilisation d'une levure active adaptée aux acides organiques faibles et une telle levure.

L'invention concerne encore plus particulièrement l'utilisation de ladite levure active adaptée aux acides organiques faibles ou d'un probiotique comprenant une telle levure, chez la volaille ou chez les ruminants.

Concernant la volaille, ladite levure ou probiotique est notamment utilisé(e) pour améliorer les performances zootechniques de la volaille.

Concernant les ruminants, ladite levure ou probiotique est notamment utilisé(e) pour une utilisation dans la prévention et/ou le traitement des troubles digestifs du ruminant, et notamment dans la prévention de l'acidose chez le ruminant.

### ARRIÈRE PLAN TECHNIQUE

### Concernant la volaille

Durant de nombreuses années, les antibiotiques qui, à des doses non médicamenteuses, ont un effet de facteur de croissance, ont été couramment utilisés pour améliorer les performances zootechniques dans l'élevage de volaille. Cependant, une telle utilisation en routine n'est pas sans conséquences, par exemple sur le développement des résistances aux antibiotiques de la part des bactéries. Aussi, en 2006, l'Union Européenne a-t-elle décidé d'interdire l'utilisation d'antibiotiques chez la volaille comme facteur de croissance, pour limiter l'apparition de phénomènes de résistance.

Les industriels ont donc cherché des alternatives aux antibiotiques. C'est ainsi que l'utilisation des probiotiques en volaille s'est étendue. La plupart des probiotiques commercialisés pour la volaille sont d'origine bactérienne principalement de la famille des *Bacillus,* compte tenu de leur capacité à sporuler et ainsi à résister aux contraintes thermiques élevées de la fabrication des aliments avicoles.

S'il a été constaté que les levures, notamment *Saccharomyces cerevisiae,* peuvent avoir un effet positif sur la croissance et les performances d'élevage des porcins et des bovins, leur impact sur la croissance et le gain de poids des volailles n'a pas été démontré et était souvent très limité et non significatif.

Par exemple, dans US2002/0146399, est proposé un produit pouvant être ajouté à l'alimentation animale notamment pour la volaille, comprenant de l'acide sorbique et au moins une culture de micro-organismes ayant une activité probiotique, telle que, par exemple, une culture de la levure *Saccharomyces cerevisiae.*

Dans WO97/00017 est proposé un probiotique, notamment utile pour des poules, contenant de la farine d'algues, de la levure sèche active et un composant minéral, la levure pouvant appartenir à l'espèce *Saccharomyces cerevisiae.*

En outre il n'existe actuellement dans l'Union Européenne aucune autorisation de levure comme probiotique pour les volailles.

Il existe donc un réel besoin de trouver des « probiotiques levures » efficaces permettant d'améliorer les performances de croissance, de gain de poids et l'indice de consommation (IC) de la volaille.

On entend par « probiotique levure », un probiotique comprenant une levure ou encore une levure qui a une fonction de probiotique. On peut également désigner le « probiotique levure » par « levure probiotique ».

### Concernant les ruminants

L'acidose, courante chez les ruminants, constitue une des préoccupations majeures de la nutrition moderne des animaux ruminants. En effet, l'accroissement des potentiels de production a entraîné l'apport de rations plus concentrées en énergie. De ce fait le rumen (la panse) des animaux doit traiter des quantités accrues de matières organiques fermentescibles et les fermentations plus intenses consécutives entraînent un état d'acidose aux effets zootechniques défavorables : interactions digestives négatives, dégradation du taux butyreux du lait, pathologies digestives et métaboliques etc.

L'acidose peut se résumer comme un trouble de l'équilibre acido-basique dû à une baisse de pH dans le rumen (pH ruminal), qui résulte de la dégradation des glucides fermentescibles dont la digestion rapide par les bactéries du rumen produit des Acides Gras Volatils (AGV) et de l'acide lactique.

Dans des conditions normales, les AGV et l'acide lactique sont absorbés par les papilles ruminales pour aider l'animal à produire du lait. L'acidose s'installe lorsque la production des AGV et d'acide lactique excède la capacité des papilles ruminales à les absorber.

Afin de contrer cet état d'acidose chez le ruminant, et notamment chez la vache laitière, différents additifs alimentaires ont été proposés dans la littérature, dont les levures probiotiques. Les levures probiotiques sont en effet reconnues pour leur rôle stabilisateur du milieu ruminai. Plusieurs études^{1,2} ont démontré l'effet des levures vivantes sur le pH ruminai même si ce dernier est fortement dépendant du régime alimentaire et en particulier de la nature même des aliments qui le composent. Chez la vache laitière à haut niveau de production, la supplémentation des rations avec les levures vivantes est une solution intéressante pour limiter les effets négatifs de l'utilisation de régimes acidogènes sur la digestion et les performances des ruminants.

Outre la mesure du pH ruminai, la mesure du potentiel d'oxydo-réduction (potentiel redox) ruminai (Eₕ en mV) s'est révélée être un outil clé pour comprendre le mode d'action des levures vivantes³. Le potentiel redox est un paramètre physico-chimique d'intérêt pour la compréhension, non seulement du fonctionnement du rumen mais aussi de l'action ruminale de la levure probiotique. Il a ainsi été montré dans la littérature⁴ que la régulation du Eₕ ruminai par les levures vivantes est particulièrement effective lorsque le risque de dysfonctionnement du rumen est suffisamment élevé⁴.

Cependant, il existe toujours à ce jour le besoin de trouver des solutions efficaces pour prévenir et/ou traiter l'acidose chez les ruminants.

### RESUME DE L'INVENTION

Les Inventeurs ont développé et mis au point de nouvelles levures actives aux propriétés particulières, en ce sens qu'elles sont notamment adaptées aux acides organiques faibles. Ainsi, selon un premier aspect, la présente invention concerne ces nouvelles levures actives adaptées aux acides organiques faibles.

Selon un deuxième aspect, l'invention concerne l'utilisation de ces levures actives adaptées chez la volaille ou chez le ruminant.

Selon un autre aspect, l'invention concerne un probiotique pour volaille ou pour ruminant caractérisé en ce qu'il comprend une levure active adaptée aux acides organiques faibles. Les Inventeurs ont également mis au point, un aliment pour volaille ou pour ruminant comprenant le probiotique selon l'invention.

Selon un autre aspect, la présente invention concerne l'utilisation d'une levure active adaptée aux acides organiques faibles pour améliorer les performances zootechniques de la volaille.

On entend, au sens de la présente invention, par « amélioration des performances zootechniques de la volaille », une amélioration des performances de croissance, de gain de poids et l'indice de consommation (IC) de la volaille.

Selon un autre aspect, la présente invention concerne une levure active adaptée aux acides organiques faibles ou un probiotique la comprenant, pour une utilisation dans la prévention et/ou le traitement des troubles digestifs du ruminant, et en particulier dans la prévention de l'acidose chez le ruminant.

Selon un autre aspect, la présente invention concerne l'utilisation d'une levure active adaptée aux acides organiques faibles dans un aliment pour volaille ou pour ruminant.

De façon avantageuse, l'utilisation d'une levure adaptée ou d'un probiotique selon l'invention permet d'éviter l'adjonction d'enzymes dans l'alimentation.

### DESCRIPTION DETAILLEE

La présente invention a pour objet une levure active *Saccharomyces cerevisiae* adaptée aux acides organiques faibles caractérisée en ce qu'elle est issue d'une souche choisie parmi les souches déposées à la CNCM respectivement sous les numéros I-4407 le 2 décembre 2010, I-5128, I-5129, I-5130, I-5131 le 31 août 2016, I-5222, I-5223 le 30 août 2017, et en ce que le rapport entre l'activité selon le test A1 de ladite levure active adaptée issue d'une souche choisie parmi I-4407, I-5128, I-5129, I-5130, I-5131, I-5222, I-5223, et l'activité selon le test A1 de la levure active non adaptée issue de la même souche choisie parmi I-4407, I-5128, I-5129, I-5130, I-5131, I-5222, I-5223, est supérieur ou égal à 1.

Le terme « levure » désigne des levures obtenues par culture d'une souche de levure.

La culture de la souche de levure est effectuée dans un milieu de culture approprié à la croissance de la souche de levure.

L'homme du métier est capable de déterminer quelle est la composition d'un milieu de culture approprié à une souche de levure donnée selon le protocole décrit dans le livre de référence « Yeast technology » par Gerald Reed et Tilak W. Nagodawithana (ISBN 0-442-31892-8) aux pages 284 à 293.

Un procédé de culture d'une souche et d'obtention d'une levure adaptée à un acide organique faible est décrit dans l'exemple 1.

Le terme « levure active », qui est synonyme de levure vivante, désigne une population de cellules de levure qui sont métaboliquement actives.

Par « levure active ... issue d'une souche », on entend une levure obtenue par culture et adaptation d'une souche.

Par « levure active adaptée aux acides organiques faibles », on entend une levure dont la tolérance à un acide organique faible donné est augmentée grâce à une précédente exposition à cet acide (Warth, 1988, Appl. Environ. Microbiol., 54 (8) : 2091-2095). La croissance et la capacité fermentaire de la levure adaptée ne sont ainsi pas inhibées lorsque la levure est en présence d'un acide organique faible pour au moins la seconde fois.

Au sens de la présente invention, les termes « levure active adaptée aux acides organiques faibles », « levure adaptée aux acides organiques faibles », « levure adaptée », « levure active adaptée » ont la même signification.

Par « acides organiques faibles », on entend les acides organiques en tant que tels et leurs sels. Ils sont choisis dans le groupe comprenant l'acide propionique, l'acide benzoïque, l'acide acétique, l'acide butyrique, l'acide citrique, l'acide formique, l'acide fumarique, l'acide lactique, l'acide malique, l'acide sorbique et leurs sels, notamment les sels de calcium ou de sodium.

A titre d'exemples plus particuliers, on citera les levures adaptées aux sels de l'acide propionique ou benzoïque, et de préférence au propionate de calcium ou au benzoate de sodium.

Un procédé d'obtention d'une levure adaptée à un acide organique faible est décrit dans l'exemple 1, basé sur le procédé décrit dans le brevet US 4 318 991.

L'adaptation des levures selon l'invention à la présence d'un acide organique faible est plus particulièrement effectuée lors des dernières heures de leur dernier stade de multiplication, par ajout de 0,1 g à 10 g d'acide organique faible et/ou leurs sels, par litre de milieu de culture.

Selon un mode de réalisation particulier de l'invention, la levure active *Saccharomyces cerevisiae* est plus particulièrement *Saccharomyces cerevisiae var. boulardii* également appelée *Saccharomyces boulardii.*

De façon avantageuse, la présente invention a pour objet une levure active adaptée, ou un probiotique la comprenant, dont le rapport de l'activité selon le test A1 (décrit ci-après) entre une levure dite « adaptée » et une levure non adaptée est supérieur ou égal à 1.

Ainsi, selon la présente invention, la levure active sélectionnée est une levure active, du genre *Saccharomyces,* qui se caractérise par un rapport de l'activité selon le test A1 entre cette levure active sélectionnée dite « adaptée » et la levure non adaptée, comme suit :

### Activité de la levure adaptée selon le test A1

Activité de la levure non adaptée selon le test A1 supérieur ou égal à 1.

Ledit test A1 correspond au protocole suivant :
on mesure pendant 2 heures le volume de CO₂ dégagé par la levure active avec le fermentomètre de Burrows et Harrisson suivant la méthode standard décrite aux pages 579 à 584 du livre de référence « Guide pratique d'analyses dans les industries de céréales » coordonné par B.Godon et W.Loisel (Ed Lavoisier - Tec & Doc - 1997 - ISBN. 2-7430-0123-2 - 2° édition) en utilisant le mode opératoire adapté suivant :
(a) Peser des lots de 20 g de farine, la farine étant une farine de froment ayant un temps de chute Hagberg de 200 à 300 secondes (tel que défini dans aux pages 680 et 681 du livre de référence cité ci-dessus), et les mettre dans des tubes propres et secs, puis ajouter dans chacun des tubes 2 g de saccharose et faire incuber ces tubes dans le râtelier du bain-marie, qui se trouve à 30°C, pendant au moins 30 minutes avant de commencer l'essai ;
(b) Prendre comme échantillon de levure l'équivalent de 1,066 g de matière sèche levure ;
(c) Préparer les échantillons délayés de levure dans 100 ml d'eau distillée (la méthode pour levure sèche est décrite à la page 583 dudit livre de référence) et ajouter 0,5 ml d'acide acétique à 6,6% (p/v) juste avant l'ajustement à 100 ml ;
(d) Pipeter les 15 ml de l'échantillon délayé bien mélangé nécessaires au malaxage, ces 15 ml apportant 1,066 g x 0,15 = 0,160 g de matières sèches levure ; et suivre pour le reste les instructions du mode opératoire décrit dans le livre de référence, en réalisant la mesure sur 2 heures.

Afin de tenir compte de l'effet du séchage sur l'activité de la levure, l'activité de la levure active selon le test A1 est définie comme suit :
- la levure testée est sous forme sèche, c'est-à-dire à une teneur en matières sèches d'au moins 90%, son activité correspond au volume de CO₂ mesuré.

Selon un autre mode de réalisation particulier de l'invention, la levure adaptée aux acides organiques faibles, comprise de préférence dans un probiotique pour volaille ou ruminant, est obtenue par culture et adaptation d'une souche *Saccharomyces cerevisiae* choisie parmi les souches déposées auprès de la Collection Nationale de Cultures de Microorganismes, 25 rue du Docteur Roux 75724 Paris cedex 15, respectivement sous les numéros CNCM I-5128, I-5129, I-5130 et I-5131, le 31 août 2016, sous les numéros CNCM I-5222 et I-5223, le 30 août 2017 et sous le numéro CNCM I-4407 le 2 décembre 2010.

Les Inventeurs ont en effet découvert de manière surprenante que des levures ayant subi un processus d'adaptation aux acides organiques faibles lors de leur culture sont plus efficaces chez la volaille ou chez le ruminant que des levures n'ayant pas subi ce processus.

Ces levures dites « adaptées » permettent ainsi notamment d'améliorer les performances de croissance, de gain de poids, et l'indice de conversion de la consommation de l'aliment par rapport au gain de poids de la volaille.

On entend par « indice de conversion de la consommation de l'aliment par rapport au gain de poids » de la volaille, l'indice de consommation de la volaille. L'indice de consommation (IC) est la quantité d'aliment ingéré (en kilogrammes) par une volaille pour prendre 1 kilogramme de poids vif.

De façon surprenante, chez le ruminant, les levures actives adaptées de l'invention permettent notamment d'améliorer la régulation de paramètres physico-chimiques du rumen tels que le pH ou le potentiel redox, et ainsi de prévenir et/ou traiter l'acidose du ruminant.

Selon un autre mode de réalisation particulier de l'invention, la levure adaptée aux acides organiques faibles est sous forme sèche.

On entend par levure sèche, toute levure ayant un taux de matière sèche supérieur à 90%.

La levure sèche active est une levure vivante, séchée de façon à conserver son pouvoir fermentaire et lui assurer une conservation très longue. Ladite levure sèche active présente une teneur élevée en cellules vivantes de levure et peut se présenter sous différentes formes, par exemple sous forme de sphérules, de granules ou de poudre, toutes ces formes ayant une teneur moyenne en eau comprise entre 4 et 8%.

Le séchage est effectué selon toute méthode connue de l'homme du métier et compatible avec la viabilité des cellules, c'est-à-dire permettant de conserver une teneur élevée en cellules vivantes. L'homme du métier sait également que, selon le type de séchage effectué, la teneur en cellules vivantes est plus ou moins élevée, et les performances dans le test A1 peuvent être affectées.

Le poids de matière sèche est déterminé par les méthodes classiques bien connues de l'homme du métier.

Selon encore un autre mode de réalisation de l'invention, la levure active adaptée se présente sous forme de granulés, de microsphérules, de farine ou sous forme hydrodispersible.

La présente invention a encore pour objet un probiotique comprenant une levure active adaptée aux acides organiques faibles telle que définie ci-dessus.

Par « probiotique », on entend des micro-organismes vivants qui, lorsqu'ils sont ingérés en quantité suffisante, exercent des effets positifs sur la santé, au-delà des effets nutritionnels traditionnels.

Le probiotique tel que défini ci-dessus comprend au moins 90% en masse de la matière sèche de la levure active adaptée.

Le probiotique de l'invention est plus particulièrement un probiotique pour volaille ou pour ruminant.

Selon un autre aspect, la présente invention concerne un aliment pour volaille ou ruminant comprenant le probiotique décrit ci-dessus.

Le probiotique et l'aliment pour volaille ou ruminant selon l'invention peuvent également comprendre un ou plusieurs ingrédients autres que ladite levure active adaptée, comme notamment des vitamines, des minéraux alimentaires, des oligo-éléments, des enzymes alimentaires, des acidifiants, des extraits végétaux, des parois de levure et des graisses alimentaires, ainsi que, le cas échéant, d'autres micro-organismes à effet probiotique.

Selon les connaissances de l'homme du métier de l'élevage de volailles, la période de croissance d'un poulet est comprise de la naissance à 35 - 42 jours. Durant ce laps de temps, il passe d'une masse de 30 à 50 grammes à une masse de 2 à 3 kilogrammes, avec une masse qui est multipliée par 5 à 6 en 10 jours. Son alimentation, en termes de quantité, est fixée préférentiellement en fonction de son âge.

Selon un mode de réalisation particulier de l'invention et sur cette base de poids de la volaille, l'animal reçoit entre 10⁶ et 10¹¹ CFU de levure active adaptée aux acides organiques faibles par kilogramme d'aliment par jour, de préférence entre 10⁹ et 10¹¹ CFU/kg d'aliment/jour, et encore plus de préférence entre 10⁹ et 10¹⁰ CFU/kg d'aliment/j our.

Le terme CFU ou UFC signifie unité formant colonie, un CFU ou UFC correspond à une colonie.

Selon un autre aspect, l'invention concerne une méthode pour améliorer les performances zootechniques de l'animal, notamment pour améliorer les performances de croissance, de gain de poids et l'indice de consommation de la volaille, consistant à fournir à l'animal une levure active adaptée aux acides organiques faibles, notamment entre 10⁶ et 10¹¹ CFU de levure active adaptée aux acides organiques faibles par kilogramme d'aliment et par jour, de préférence entre 10⁹ et 10¹¹ CFU/kg d'aliment/jour, et encore plus de préférence entre 10⁹ et 10¹⁰ CFU/kg d'aliment/jour.

Selon un autre aspect, l'invention concerne l'utilisation d'une levure active adaptée aux acides organiques pour améliorer les performances zootechniques de la volaille.

Par « performances zootechniques », on entend les performances de croissance, le gain de masse ou de poids de l'animal, l'indice de consommation, et les paramètres de reproduction des animaux.

Il a en effet été constaté que l'utilisation d'une levure adaptée chez la volaille permettait d'augmenter le gain de poids, la quantité d'aliment ingéré et d'améliorer l'indice de consommation de l'aliment par rapport au gain de poids, en particulier chez la volaille en croissance.

L'invention a plus particulièrement pour objet l'utilisation d'une levure active adaptée telle que définie ci-dessus ou d'un probiotique tel que défini ci-dessus, chez la volaille, notamment pour améliorer les performances de croissance, de gain de poids et l'indice de consommation (IC) de la volaille.

L'invention a également pour objet l'utilisation d'une levure active adaptée telle que définie ci-dessus ou d'un probiotique tel que défini ci-dessus, chez le ruminant.

Plus particulièrement une telle utilisation chez le ruminant permet d'agir sur les paramètres physico-chimiques du rumen tels que le pH, le potentiel redox, mais également sur les paramètres fermentaires tels que la teneur en AGV présents dans le rumen.

Selon un mode de réalisation avantageux de l'invention, l'utilisation de la levure active adaptée permet de maintenir et/ou de rétablir le pH du rumen à une valeur supérieure ou égale à 5,8 ².

Selon un autre mode de réalisation avantageux, l'utilisation de la levure active adaptée de l'invention permet de maintenir et/ou de rétablir le potentiel redox du rumen à une valeur allant de - 200 mV à - 150 mV.

Les valeurs de pH et de potentiel redox ci-dessus décrites correspondent à celles obtenues par la méthode de mesure décrite dans l'exemple 4 ci-après.

Selon encore un autre mode de réalisation avantageux, l'utilisation de la levure active adaptée de l'invention permet d'augmenter la concentration en AGV du rumen.

En agissant sur les paramètres physico-chimiques ou fermentaires tels que ci-dessus décrits, la levure active adaptée de l'invention permet avantageusement d'agir sur l'acidose du ruminant, et plus particulièrement de prévenir l'acidose ruminale chez le ruminant.

L'invention a ainsi encore pour objet une levure active adaptée telle que définie ci-dessus ou un probiotique tel que défini ci-dessus, pour une utilisation dans la prévention et/ou le traitement des troubles digestifs du ruminant, et plus particulièrement dans la prévention de l'acidose chez le ruminant.

Selon un autre aspect, l'invention concerne une méthode pour prévenir et/ou traiter l'acidose du ruminant, consistant à fournir à l'animal une levure active adaptée aux acides organiques faibles, notamment en une quantité allant de 0,5 g à 50 g de levure active adaptée par animal et par jour, de préférence de 1 g à 10 g de levure adaptée par animal et par jour.

Selon un autre aspect, l'invention concerne l'utilisation d'une levure active adaptée aux acides organiques dans un probiotique et/ou dans un aliment pour volaille ou ruminant tels que décrits ci-dessus.

L'invention a pour objet l'utilisation d'une levure active adaptée telle que définie ci-dessus ou d'un probiotique tel que défini ci-dessus, dans un aliment pour volaille ou pour ruminant.

Selon un autre mode de réalisation particulier de l'invention, la levure active adaptée aux acides organiques et/ou le probiotique sont mis en oeuvre sous une forme adaptée à une utilisation dans une matrice alimentaire, ladite matrice étant par exemple des granulés, de la farine, ou de l'eau de boisson.

La levure adaptée aux acides organiques faibles et/ou le probiotique sont par exemple sous forme de granulés ou de farine lorsqu'ils sont mélangés à l'aliment et sous forme hydrodispersible, lorsqu'ils sont mélangés à l'eau de boisson.

Selon un autre aspect, l'invention concerne l'utilisation d'une levure active adaptée aux acides organiques faibles dans laquelle l'animal, en particulier une volaille pesant entre 30 et 3000 g, reçoit entre 10⁶ et 10¹¹ CFU de levure active adaptée aux acides organiques faibles par kilogramme d'aliment par jour, de préférence entre 10⁹ et 10¹¹ CFU/kg d'aliment/jour, et encore plus de préférence entre 10⁹ et 10¹⁰ CFU/kg d'aliment/jour.

Le probiotique, l'aliment, l'utilisation de la levure adaptée et la levure adaptée suivant l'invention sont notamment utiles dans le domaine de l'élevage de volailles choisies dans le groupe comprenant :
- les oiseaux de basse-cour et gallinacées, et notamment les poules, y compris les poussins, les coqs et coquelets, les chapons et les poules pondeuses, les dindes et les dindons,
- les ansériformes et notamment les canards, les canes, les oies, et les faisans, les pintades, les cailles et les perdrix,
- les colombins, notamment les pigeons,
- les ratites, notamment les autruches, les émeus et les kiwis.

Selon un autre aspect, l'invention concerne l'utilisation d'une levure active adaptée aux acides organiques faibles dans laquelle le ruminant reçoit de 0,5 g à 50 g de levure active adaptée aux acides organiques faibles par jour, et de préférence entre 1 g et 10 g de levure par jour.

Le probiotique, l'aliment, l'utilisation de la levure adaptée et la levure adaptée suivant l'invention sont notamment utiles dans le domaine de l'alimentation des ruminants de la famille des bovidés et des camélidés.

Les bovidés (Bovidae) comprennent plusieurs sous-familles, dont en particulier les bovinés (dont font partie les bovins) et les caprinés (qui englobent les caprins et les ovins).

Les camélidés (Camelidae) sont des mammifères artiodactyles engobant par exemple le dromadaire, le chameau ou le lama.

Le probiotique suivant l'invention comprend au moins 90% en masse de matière sèche de la levure active sélectionnée dite « adaptée ».

La présente invention va être illustrée à l'aide des exemples de réalisation qui suivent, étant entendu que ceux-ci n'ont aucune portée limitative.

### EXEMPLES

### Exemple 1 : Procédé d'obtention d'une levure active sèche adaptée à un acide organique faible

### 1.1.Procédé de culture « standard »

Les levures peuvent être obtenues par une culture sur mélasse de betteraves ou de cannes par exemple, suivant le procédé discontinu alimenté (en anglais : fed-batch).

Le procédé de culture comporte une succession d'étapes de fermentation comme décrit aux pages 284 à 293 du livre de référence « Yeast technology » par Gerald Reed et Tilak W. Nagodawithana (ISBN 0-442-31892-8).

### 1.2.Procédé pour induire une adaptation

Le procédé pour induire l'adaptation à un acide organique faible correspond au procédé de culture standard ci-dessus, modifié comme il suit selon les étapes suivantes :
1. Acidification : 2 à 3 h avant la fin de la culture (cf. ci-dessus le procédé de culture « standard ») et avant la phase d'adaptation, ajuster le pH du milieu de culture à une valeur comprise entre 4,0 et 5,5 et le maintenir jusqu'à la fin de la phase d'adaptation, par exemple avec de l'acide sulfurique ;
2. Adaptation : ajouter dans le milieu de culture une quantité d'acide organique faible ou de son sel associé de façon à obtenir entre 1500 et 3000 ppm (partie par million) d'acide organique faible en poids dans le milieu de culture. La dose d'ajout d'acide faible est liée à la composition de l'acide faible ou de son sel associé utilisé. Le milieu de culture est dépourvu de tampon.

L'homme du métier saura ajuster le pH et la quantité d'acide organique faible à utiliser sans déployer des efforts excessifs.

### 1.3.Séchage

Les levures sèches actives sélectionnées, c'est-à-dire levures sèches présentant une teneur élevée en cellules vivantes de levure, sont obtenues par séchage selon une technique connue de l'homme du métier pour obtenir un taux de matière sèche supérieur à 90%.

### Exemple 2 : Mesure du rapport de l'activité selon le test A1 entre une levure adaptée et une levure non adaptée

Les levures a et b correspondent à 2 lots différents a et b de la souche de levure non adaptée déposée à la CNCM le 31 août 2016 sous le numéro I-5131, c'est-à-dire une levure n'ayant pas subi le procédé d'adaptation décrit dans l'exemple 1.2.

La levure dite « adaptée », selon le procédé décrit dans l'exemple 1.1 et 1.2, utilisée dans le test A1 correspond à 2 lots différents a et b de la même souche de levure I-5131.

Le test A1 est celui décrit *supra.*

### Résultats du test A1 :

**Tableau 1**

| Souches | Activité (ml de CO₂) |
|---|---|
| I-5131 non adaptée (a) | 103 |
| I-5131 non adaptée (b) | 113 |
| I-5131 adaptée (a) | 106 |
| I-5131 adaptée (b) | 132 |

### Rapport de l'activité selon les tests A1 entre la levure 1-5131 adaptée et la levure I-5131 non adaptée :

**Tableau 2**

| Lots | Rapport |
|---|---|
| Lot (a) | 1,029 |
| Lot (b) | 1,168 |

Conclusion : Les levures adaptées utilisées selon l'invention présentent ainsi un rapport supérieur à 1.

D'autres tests ont été effectués, tous lots confondus, sur respectivement :
- des lots de levures non adaptées issues de la souche I-5131,
- des lots de levures adaptées à l'acide propionique issues de la souche I-5131.

Les résultats obtenus sont donnés dans le tableau 3 ci-dessous :

| Souches | Activité (ml de CO₂) (moyenne) | Erreur Standard | Limite Inférieure | Limite Supérieure |
|---|---|---|---|---|
| I-5131 non adaptée | 109,571 | 2,173 | 105,140 | 114,003 |
| I-5131 adaptée à l'acide propionique | 121,667 | 1,659 | 118,282 | 125,051 |

Conclusion : les levures actives adaptées utilisées selon l'invention présentent un rapport supérieur à 1, tous lots confondus.

D'autres tests ont été effectués, tous lots confondus, sur respectivement :
- des lots de levures non adaptées issues de la souche I-5129,
- des lots de levures adaptées à l'acide propionique issues de la souche I-5129. Les résultats obtenus sont donnés dans le tableau 4 ci-dessous :

| Souches | Activité (ml de CO₂) (moyenne) |
|---|---|
| I-5129 non adaptée | 112,3 |
| I-5129 adaptée à l'acide propionique | 126,3 |

Conclusion : les levures actives adaptées utilisées selon l'invention présentent un rapport supérieur à 1, tous lots confondus.

### Exemple 3 : Essais d'une levure dite adaptée, pour l'alimentation de la volaille

Les levures sèches actives adaptées présentent des propriétés avantageuses surprenantes dans le domaine de l'aviculture.

Ces propriétés avantageuses surprenantes sont illustrées par les résultats du test suivant sur des poussins.

Classiquement, selon les connaissances de l'homme du métier de l'élevage de volailles, l'alimentation utilisée pour les poussins est un blé viscosant, autrement dit un blé de qualité moyenne voire mauvaise. Ceci représente un challenge nutritionnel, autrement dit une sorte de stress. Selon les connaissances de l'homme du métier, l'un des moyens d'améliorer la digestibilité d'un tel blé est l'adjonction d'enzymes. Dans le cadre de la présente invention, il n'y a pas d'adjonction d'enzymes, ce qui représente un avantage non négligeable en termes de coût.

Dans ce test, les résultats obtenus par l'adjonction d'un probiotique selon l'invention à un aliment farine pour poussins sont comparés avec les résultats obtenus avec cet aliment sans additif, et avec un mélange de cet aliment avec la même levure sèche active non adaptée.

### Poussins

Le test a été effectué sur une période de 1 à 21 jours sur une population de poussins répondant aux critères suivants : poulets ROSS âgés au début de l'essai de 1 jour.

### Composition de l'aliment farine pour poussins

L'aliment farine consiste principalement en du blé et du maïs sans enzymes anti-viscosantes (comme expliqué ci-dessus) selon la composition suivante :

**Tableau 5**

| **Ingrédient** | **Quantité (%)** |
|---|---|
| Blé | 40,0 |
| Maïs | 39,8 |
| Farine de soja | 16,6 |
| DL méthionine | 0,1 |
| Di-phosphate de calcium | 1,5 |
| Carbonate de calcium | 1,0 |
| Prémélange vitamines - minéraux | 0,6 |
| Sel | 0,4 |

La composition des différents mélanges pour poussins est donnée dans le tableau 6 ci-dessous.

**Tableau 6**

| Mélange testé | Additif ajouté par g d'aliment farine |
|---|---|
| Témoin | / |
| Mélange levure non adaptée | 800 g/T soit 10⁷ CFU/g d'aliment de la levure sèche active I-5128 |
| Mélange levure adaptée | 800 g/T soit 10⁷ CFU/g d'aliment de la levure sèche active adaptée I-5128 |

### Protocole du test

La population de poussins a été divisée de manière aléatoire en 2 réplicats de 3 lots de 6 cages contenant chacune 20 poussins, chaque lot de poussins recevant un mélange à tester selon le tableau 5.

A partir du premier jour, les poussins de chaque groupe ont été alimentés *ad libitum* avec le mélange correspondant au lot d'essai. Les poussins disposaient également *ad libitum* d'eau.

Aucune autre source d'alimentation n'était mise à la disposition des poussins.

La quantité de farine consommée par chaque groupe était mesurée.

A différents moments durant le test, le poids vivant des poussins était mesuré, respectivement à J0 (poulet de 1 jour), J+7 j ours, J+ 14 jours J+21 jours.

### Résultats :

Les résultats du test, à J+21 jours, sont donnés dans le tableau 7, dans lequel :
- FI-x signifie la quantité totale moyenne (en g) de mélange consommé par poussin du groupe donné après x j ours, en l'occurrence ici FI-21, après 21 jours ;
- LW-x signifie le poids vivant moyen (en g) des poussins du groupe donné après x jours, en l'occurrence ici LW-21 après 21 jours.
- FCR-x signifie le rapport entre, d'une part, le gain de poids vivant moyen (en g) des poussins du groupe donné après x jours et, d'autre part, la quantité totale moyenne de mélange consommé par poussin dudit groupe après x jours, en l'occurrence ici FCR-21 après 21 jours.

| | **Control** | **I-5128** | **I-5128 adaptée** | **SEM** | **ANOVA (P)** |
|---|---|---|---|---|---|
| Initial LW | 40 | 40 | 40 | | |
| LW-21 | 516,2a | 514,6a | 545,2b | 6,9 | **0,036** |
| FI-21 | 820,9 | 814,3 | 833,6 | 7,8 | 0,551 |
| FCR-21 | 1,67a | 1,65a | 1,60b | 0,01 | **0,021** |

SEM signifie standard error of the mean, appellation anglo-saxonne de l'écart type de la moyenne.

ANOVA signifie ANalysis Of VAriance

Les différentes lettres utilisées a, b indiquent si les valeurs sont statistiquement différentes ou non. Ainsi les résultats ne sont pas significativement différents lorsque la même lettre est reportée (p > 0,05).

Conclusion : Les résultats montrent que la supplémentation avec une levure adaptée améliore significativement les résultats de croissance (LW21) et de conversion alimentaire (FCR21) des poulets alors que la levure non adaptée n'a pas d'effet.

### Exemple 4 : Influence d'une levure active adaptée sur les paramètres physico-chimiques et fermentaires des ruminants

### Description du protocole/ méthodologie

L'outil *in vitro* choisi dans cet exemple est la méthode « Dual-Flow » (« double flux ») qui a démontré sa capacité à imiter efficacement la fermentation dans le rumen^{6,7}.

Dans un système traditionnel à double flux, le pH est maintenu constant à l'aide d'acide et de base. Dans cet exemple, le pH n'est contrôlé que par la quantité et le pouvoir tampon de la salive artificielle injectée dans les réacteurs de fermentation, de sorte que les paramètres physico-chimiques de la cinétique évoluent de la même façon que s'ils étaient mesurés dans le rumen.

Dans le système de fermentation à double flux utilisé pour mesurer les paramètres de l'invention, le taux de dilution en liquide et le taux de dilution en solides peuvent être configurés par l'utilisateur pour mimer un rumen en situation de faible ou forte acidose.

Il est composé de 24 bioréacteurs de 1 L équipés de 2 bras latéraux pour évacuer les particules solides et pour alimenter et pomper le liquide. Chaque bioréacteur possède 4 entrées, à savoir, une alimentation de la salive, une sortie de gaz, une sonde de pH et une sonde redox.

La température est maintenue à 39°C en incubant le bioréacteur contenant du liquide ruminal dans un bain-marie et le contenu est agité à l'aide d'un agitateur.

Le pH est maintenu avec un tampon bicarbonate⁸.

Le pH et le potentiel redox (Eₕ) sont enregistrés en temps réel avec un enregistreur de données à entrées multiples

Après l'inoculation, le système est hermétiquement fermé et l'état anaérobie est évalué en mesurant le potentiel redox. Les effluents sont collectés quotidiennement.

Des vaches en lactation portant une canule au niveau du rumen ont été échantillonnées et le liquide a été recueilli, filtré à travers 2 couches de mousseline et mélangé avec un volume égal de tampon bicarbonate dans des conditions anaérobies.

Chaque bioréacteur est ensuite rempli jusqu'à ce qu'il déborde tout en étant soumis à du CO₂ et inoculé avec 15 g d'aliment composé d'ensilage de maïs, de tourteaux de soja, de foin et de maïs transformé.

L'alimentation (15 g de granulés) de chaque bioréacteur s'effectue quotidiennement à 09 heures à 16 heures.

Initialement, les expériences à double flux sont conçues conformément à ce qui est décrit dans Stem et al⁸.

Des essais antérieurs ont révélé qu'une adaptation à l'aliment d'au moins 7 jours est nécessaire pour obtenir un schéma de fermentation stable.

Les souches de levure vivantes I-5129 non adaptées et adaptées au propionate de calcium ou au benzoate de sodium ont été évaluées en UFC iso-équivalentes Le nombre d'UFC a été ajusté pour avoir des UFC iso-équivalentes.

Des paramètres physico-chimiques tels que le pH, le potentiel redox (Eh) ont été mesurés et organisés sur la cinétique de mesure établie lors du seizième et dix-septième jour de fermentation corrigée par la période de covariable lors du sixième et septième jour de mesure.

L'analyse des AGV individuels et totaux a été effectuée sur le fluide du rumen (liquide ruminal) prélevé sur les mêmes bases que les mesures de pH et de potentiel redox.

Des enregistrements diurnes du pH et de l'En ont été réalisés au cours des différentes phases (phases de stabilisation et d'adaptation) à l'aide du logiciel de mesure en continu. Les données ont été collectées et traitées à l'aide du modèle mixte linéaire par le logiciel SPSS (IL, Chicago) et les paramètres de fermentation ont été traités à l'aide du modèle univarié.

Chaque fermenteur individuel a été considéré comme une unité expérimentale.

Pour chaque traitement (levure ou pas de levure), il y avait 6 répétitions.

Les traitements sont montrés significativement différents à P <0,05 et les tendances à P <0,10 ainsi que la comparaison par paires.

### Résultats

Dans les tableaux ci-dessous :
- « I-5129 NA » désigne la levure non adaptée,
- « I-5129 Prop » désigne la levure adaptée au propionate de calcium,
- « I-5129 Benz » désigne la levure adaptée au benzoate de sodium.

Le contrôle représente un échantillon du liquide ruminal sans présence de levure.

### pH du rumen

| | Contrôle | I-5129 NA | I-5129 Prop |
|---|---|---|---|
| **pH** | 5,62a | 5,60a | 5,84b |
| Moyenne sur 24 heures | | | |

a,b : les valeurs moyennes sur une même ligne affectée d'un même suffixe ne sont pas statistiquement différentes entre elles.

L'effet pH est notable sur la levure adaptée au propionate de calcium.

### Potentiel redox (Eₕ) du rumen

| | Contrôle | I-5129 NA | I-5129 Prop | I-5129 Benz |
|---|---|---|---|---|
| **Eₕ (mV)** | -163,8b | -166,5b | -172,7a | -172,1a |
| Moyenne sur 24 heures | | | | |

a,b : les valeurs moyennes sur une même ligne affectées d'un même suffixe ne sont pas statistiquement différentes entre elles.

La cinétique du potentiel redox montre une chute significative vers des valeurs inférieures avec les levures adaptées I-5129 au propionate et au benzoate par rapport au contrôle (-10mV) et à la levure non adaptée (-6mV).

La cinétique du potentiel redox Eₕ pour les levures adaptées a montré une différence significative (-6 mV) par rapport aux levures non adaptées.

| | Contrôle | I-5129 NA | I-5129 Prop |
|---|---|---|---|
| **Eₕ (mV)** Moyenne sur 24 heures | -156,8b | -147,5b | -166,0a |

a,b : les valeurs moyennes sur une même ligne affectées d'un même suffixe ne sont pas statistiquement différentes entre elles.

Les valeurs de Eₕ varient de -147,5 mV à -166,0 mV pour les levures respectivement non adaptée et adaptée.

### Conclusion

Les levures adaptées ont montré des valeurs de potentiel redox plus faibles, ce qui montre que le pouvoir réducteur du milieu ruminai est renforcé.

### Concentrations en AGV

| | Contrôle | I-5129 NA | I-5129 Benz |
|---|---|---|---|
| Teneur totale en **AGV** (mM/L) | 90,4a | 92,4a | 93,7b |

Les concentrations totales en AGV varient de 90,4 mM pour le témoin sans levure à 93,7 mM avec la levure I-5129 adaptée au benzoate.

Les concentrations totales en AGV augmentent significativement avec la levure I-5129 adaptée au benzoate (+3,7% ou 3,3 mM/L) par rapport au contrôle ou par rapport à la levure I-5129 non adaptée (+1,4% ou 1,3 mM/L).

### Conclusion :

La levure adaptée I-5129 au benzoate favorise un milieu ruminal (rumen) plus réducteur qui se traduit par une teneur en AGV plus conséquente.

ITEM 1. Levure active *Saccharomyces cerevisiae* adaptée aux acides organiques faibles caractérisée en ce qu'elle est issue d'une souche choisie parmi les souches déposées à la CNCM respectivement sous les numéros I-4407 le 2 décembre 2010, I-5128, I-5129, I-5130, I-5131 le 31 août 2016, I-5222, I-5223 le 30 août 2017, et en ce que le rapport entre l'activité selon le test A1 de ladite levure active adaptée issue d'une souche choisie parmi I-4407, I-5128, I-5129, I-5130, I-5131, I-5222, I-5223, et l'activité selon le test A1 de la levure active non adaptée issue de la même souche choisie parmi I-4407, I-5128, I-5129, I-5130, I-5131, I-5222, I-5223, est supérieur ou égal à 1.

ITEM 2. Levure active adaptée selon ITEM 1, caractérisée en ce qu'elle est adaptée aux acides choisis dans le groupe comprenant l'acide propionique, l'acide benzoïque, l'acide acétique, l'acide butyrique, l'acide citrique, l'acide formique, l'acide fumarique, l'acide lactique, l'acide malique, l'acide sorbique et leurs sels, notamment les sels de calcium ou de sodium.

ITEM 3. Levure active adaptée selon ITEM 2, caractérisée en ce qu'elle est adaptée aux sels de l'acide propionique ou benzoïque, et de préférence au propionate de calcium ou au benzoate de sodium.

ITEM 4. Levure active adaptée selon l'un quelconque des ITEMS 1 à 3, caractérisée en ce que la levure active adaptée est sous forme sèche.

ITEM 5. Levure active adaptée selon l'un quelconque des ITEMS 1 à 4, caractérisée en ce qu'elle se présente sous forme de granulés, de microsphérules, de farine ou sous forme hydrodispersible.

ITEM 6. Probiotique caractérisé en ce qu'il comprend une levure active adaptée selon l'un quelconque des ITEMS 1 à 5.

ITEM 7. Probiotique selon ITEM 6, caractérisé en qu'il comprend au moins 90% en masse de la matière sèche de la levure active adaptée.

ITEM 8. Utilisation d'une levure active adaptée selon l'un quelconque des ITEMS 1 à 5, ou d'un probiotique selon ITEM 6 ou 7, chez la volaille, notamment pour améliorer les performances de croissance, de gain de poids et l'indice de consommation (IC) de la volaille.

ITEM 9. Utilisation d'une levure active adaptée selon l'un quelconque des ITEMS 1 à 5, ou d'un probiotique selon ITEM 6 ou 7, chez les ruminants.

ITEM 10. Levure active adaptée selon l'un quelconque des ITEMS 1 à 5, ou probiotique selon ITEM 6 ou 7, pour une utilisation dans la prévention et/ou le traitement des troubles digestifs du ruminant, et en particulier de l'acidose chez le ruminant.

ITEM 11. Utilisation d'une levure active adaptée selon l'un quelconque des ITEMS 1 à 5, ou d'un probiotique selon ITEM 6 ou 7, dans un aliment pour volaille ou pour ruminant.

ITEM 12. Utilisation selon ITEM 11, caractérisée en ce que l'animal, en particulier la volaille, reçoit entre 10⁶ et 10¹¹ CFU de levure active adaptée par kilogramme d'aliment par jour, de préférence entre 10⁹ et 10¹¹ CFU/kg d'aliment/jour, et encore plus de préférence entre 10⁹ et 10¹⁰ CFU/kg d'aliment/jour.

ITEM 13. Utilisation selon ITEM 11, caractérisée en ce que l'animal, en particulier le ruminant, reçoit de 0,5 g à 50 g de levure active adaptée par jour, de préférence de 1 g à 10 g de levure active adaptée par jour.

### Références bibliographiques

1. Marden, J.P., Julien, C., Monteils, V., Auclair, E., Moncoulon, R., Bayourthe, C., 2008, How does live yeast differ from sodium bicarbonate to stabilize ruminal pH in high yielding dairy cows? J. Dairy Sci. 91, 3528-3535.
2. Desnoyers, M., Giger-Reverdin, S., Bertin, G., Duvaux-Ponter, C., Sauvant, D., 2009, Meta-analysis of the influence of Saccharomyces cerevisiae supplementation on ruminal parameters and milk production of ruminants. J. Dairy Sci. 92(4), 1620-1632.
3. Marden, J.P., Bayourthe, C., Enjalbert, F., Moncoulon, R., 2005, A new device for measuring kinetics of ruminal pH and redox potential in dairy cows. J. Dairy Sci. 88, 277-281.
4. Pinloche, E., McEwan, N., Marden, J.P., Bayourthe, C., Auclair, E., Newbold, C.J., 2013, The effects of a probiotic yeast on the bacterial diversity and population structure in the rumen of cattle. PLoS ONE 8(7): e67824. doi:10.1371/journal.pone.0067824.
5. Huang et al., Analyse quantitative de l'effet des levures vivantes sur le potentiel redox ruminai chez la vache laitière. Rencontres autour des Recherches sur les Ruminants 23, 41.
6. Busquet M, Calsamiglia S, Ferret A, Cardozo PW, Kamel C (2005) Effects of cinnamaldehyde and garlic oil on rumen microbial fermentation in a dual flow continuous culture. J Dairy Sci 88: 2508-2516.
7. Cerrato-Sanchez M, Calsamiglia S, Ferret A (2008) Effect of the magnitude of the decrease of rumen pH on rumen fermentation in a dual-flow continuous culture system. J Anim Sci 86: 378-383.
8. M.D. Stern, H.W. Hoover (1990). The dual flow continuous culture system. Proc. Continuous Culture Fermenters: Frustration or fermentation, Northwest ADSA-ASAS Régional meeting, Chazy, NY (1990), pp. 17-32.

## Revendications

1. Levure active *Saccharomyces cerevisiae* adaptée à un acide organique faible ou à un de ses sels, pour une utilisation dans la prévention de l'acidose chez le ruminant, ladite levure adaptée présentant les caractéristiques suivantes :
- elle est obtenue par culture d'une souche de levure déposée à la CNCM sous le numéro I-5129 le 31 août 2016 ou sous le numéro I-5223 le 30 août 2017 ; par une étape d'acidification comprenant, avant la fin de la culture et avant la phase d'adaptation, l'ajustement du pH du milieu de culture à une valeur comprise entre 4,0 et 5,5 et son maintien jusqu'à la fin de la phase d'adaptation ; et par une étape d'adaptation comprenant l'ajout au milieu de culture d'une quantité d'acide organique faible ou de son sel associé de manière à obtenir entre 1500 et 3000 ppm (parties par million) d'acide organique faible en poids dans le milieu de culture ;
- le rapport de l'activité selon le test A1 entre la levure active adaptée et la levure active non adaptée est supérieur ou égal à 1, ce qui signifie que le rapport entre le volume de CO₂ dégagé par la levure active adaptée obtenue par culture de la souche de levure I-5129 ou I-5223 et le volume de CO₂ dégagé par la levure active non adaptée obtenue par culture de la même souche I-5129 ou I-5223 est supérieur ou égal à 1, lesdits volume de CO₂ étant mesurés pendant 2 heures ;
- elle présente une tolérance augmentée à un acide organique faible grâce à une précédente exposition à cet acide, de sorte que la croissance et la capacité fermentaire de ladite levure adaptée ne sont pas inhibées lorsque la levure est en présence dudit acide organique faible pour au moins la deuxième fois,
- l'acide organique faible est choisi parmi l'acide propionique, l'acide benzoïque ou un de leurs sels.

2. Levure active adaptée pour une utilisation selon la revendication 1, **caractérisée en ce que** la levure active adaptée est adaptée aux sels de calcium ou de sodium de l'acide propionique ou l'acide benzoïque.

3. Levure active adaptée pour une utilisation selon la revendication 2, **caractérisée en ce que** la levure active adaptée est adaptée au propionate de calcium ou au benzoate de sodium.

4. Levure active adaptée pour une utilisation selon l'une quelconque des revendications 1 à 3, **caractérisée en ce que** la levure active adaptée est sous forme sèche.

5. Levure active adaptée pour une utilisation selon l'une quelconque des revendications 1 à 4, **caractérisée en ce que** la levure active adaptée se présente sous forme de granulés, de microsphérules, de farine ou sous forme hydrodispersible.

6. Levure active adaptée pour une utilisation selon l'une quelconque des revendications 1 à 5, **caractérisée en ce que** la levure active adaptée est dans un probiotique pour ruminant.

7. Levure active adaptée pour une utilisation selon la revendication 6, **caractérisée en ce que** le probiotique comprend au moins 90% en masse de la matière sèche de la levure active adaptée.

8. Levure active adaptée pour une utilisation selon l'une quelconque des revendications 1 à 7, dans un aliment pour ruminants.

9. Levure active adaptée pour une utilisation selon la revendication 8, **caractérisée en ce que** le ruminant reçoit de 0,5 g à 50 g de levure active adaptée par jour, et de préférence de 1 g à 10 g de levure active adaptée par jour.
